# EUROPÄISCHE PATENTSCHRIFT

(11) **EP 3 383 250 B1**
(45) Veröffentlichungstag und Bekanntmachung des Hinweises auf die Patenterteilung: **25.09.2019**
(21) Anmeldenummer: 16795266.2
(22) Anmeldetag: 07.11.2016
(51) Int. Cl.: A61B 5/00, A61B 5/024, A61B 5/1171, B60R 25/25

(54) **ZUGANGSSYSTEM FÜR EIN KRAFTFAHRZEUG**
ACCESS SYSTEM FOR A VEHICLE
SYSTÈME D'ACCÈS POUR UNE VÉHICULE

(30) Priorität: 04.12.2015 DE 102015015741
(43) Veröffentlichungstag der Anmeldung: 10.10.2018
(73) Patentinhaber: Audi AG, 85045 Ingolstadt (DE)
(72) Erfinder: LACHER, Peter, 85080 Gaimersheim (DE); STEINHORST, Sebastian, 49219 Glandorf (DE)
(86) Internationale Anmeldenummer: PCT/EP2016/076784
(87) Internationale Veröffentlichungsnummer: WO 2017/092971

(56) Entgegenhaltungen:
- DE-A1-102009 002 906
- US-A1- 2010 148 923
- US-A1- 2013 217 979
- US-A1- 2014 081 155
- US-A1- 2015 081 169
- US-A1- 2015 087 919

## Beschreibung

Die Erfindung betrifft ein Zugangssystem für ein Kraftfahrzeug mit einem Armbandcomputer zum Tragen an einem Arm eines Benutzers.

Armbandcomputer zum Tragen an einem Arm eines Benutzers, beispielsweise in Form sogenannter Smartwatches, sind an sich bekannt. Bei derartigen Smartwatches handelt es sich üblicherweise um eine Armbanduhr, die zusätzlich über Sensoren, Aktuatoren sowie zusätzliche Computerfunktionalitäten und Konnektivitätsfunktionen verfügt. Smartwatches können häufig neben der Uhrzeit weitere Informationen darstellen und lassen sich meist über zusätzliche Programme, sogenannte Apps vom Anwender individuell mit neuen Funktionen aufrüsten.

Die CN 101114401 A zeigt beispielsweise einen Armbandcomputer zum Tragen an einem Arm eines Benutzers, welcher dazu ausgelegt ist, einen Puls des Benutzers zu erfassen und sich bei Unregelmäßigkeiten automatisch über eine Bluetooth-Verbindung mit einem Mobilfunktelefon zu verbinden, um zum Beispiel einen Notruf zu senden.

Die DE 20 2006 011 486 U1 zeigt einen GPS-Empfänger, der über eine Bluetooth-Schnittstelle GPS-Daten versenden kann. Der GPS-Empfänger verfügt über eine automatische Stromsparfunktion. Eine eingebaute Elektronik schaltet den GPS-Empfänger aus, sofern sich ein Fahrzeug, in welchem der GPS-Empfänger verbaut ist, im Stillstand befindet. Sobald sich das Fahrzeug bewegt, wird die Stromversorgung des GPS-Empfängers wieder eingeschaltet.

Die DE 103 49 165 A1 zeigt einen Kopfhörer, der von einem mit einem Pulssensor ausgestatten Brustgurt erfasste Pulsdaten drahtlos, beispielsweise über eine Bluetooth-Verbindung, empfangen kann.

Die US 2015/0087919 A1 beschreibt eine Smartwatch, welche unterschiedliche Sensoren zur Messung von Vitalparametern aufweist. Die Smartwatch kann zudem einen Sensor aufweisen, mittels welchem ermittelt werden kann, ob die Form eines Armbands der Smartwatch verändert worden ist. Basierend darauf kann bestimmt werden, ob ein Benutzer die Smartwatch angelegt hat. Sobald auf diese Weise ermittelt worden ist, dass die Smartwatch angelegt wurde, wird die Smartwatch automatisch aktiviert.

Die US 2010/0148923 A1 beschreibt ein biometrisches Authentifizierungssystem eines Kraftfahrzeugs. Das Authentifizierungssystem dient dazu, das Kraftfahrzeug zu verriegeln, entriegeln und einen Motor des Kraftfahrzeugs zu starten. Dafür nutzt das Authentifizierungssystem biometrische Daten eines Benutzers, um zu überprüfen, ob es sich um einen berechtigten Benutzer handelt. Mittels einer fahrzeugseitigen Kamera kann beispielsweise ein Gesicht des Benutzers oder eine Iris des Benutzers erfasst werden. Zudem können mittels einer in einem Türgriff oder in einem Startknopf angeordneten Sensorik Fingerabdrücke oder Informationen über Venen des Benutzers erfasst werden.

Die US 2015/0081169 beschreibt ein Steuerungs- und Zugangssystem für ein Kraftfahrzeug mit Multifaktor-Authentifizierung. Das System umfasst als ersten Authentifizierungsfaktor eine tragbare Vorrichtung, z. B. eine Smartwatch, welche einen Pulssensor oder Biometriesensor und einen digitalen Schlüssel sowie ein Funkmodul enthält. Als zweiter Authentifizierungsfaktor ist fahrzeugseitig eine Kamera oder ein Lichtsensor zur Gestenerkennung oder zur Erkennung von Skelett-Gelenk-Beziehungen vorgesehen.

Es ist die Aufgabe der vorliegenden Erfindung, einen besonders energieeffizienten Betrieb eines zum Tragen an einem Arm eines Benutzers ausgelegten Armbandcomputers zu ermöglichen.

Diese Aufgabe wird durch ein Zugangssystem für ein Kraftfahrzeug mit einem Armbandcomputer zum Tragen an einem Arm eines Benutzers mit den Merkmalen des Patentanspruchs 1 gelöst. Vorteilhafte Ausgestaltungen mit zweckmäßigen und nicht trivialen Weiterbildungen der Erfindung sind in den abhängigen Ansprüchen angegeben.

Das erfindungsgemäße Zugangssystem für ein Kraftfahrzeug umfasst einen Armbandcomputer zum Tragen an einem Arm eines Benutzers, der eine Erfassungseinrichtung aufweist, welche dazu ausgelegt ist, zumindest einen Vitalparameter des Benutzers zu erfassen. Des Weiteren umfasst der Armbandcomputer ein Funkmodul, welches im aktivierten Zustand dazu ausgelegt ist, eine drahtlose Datenverbindung mit einem Funkmodul eines anderen Objekts herzustellen und aufrechtzuerhalten. Der Armbandcomputer des Zugangssystems zeichnet sich dabei dadurch aus, dass der Armbandcomputer eine Steuereinrichtung umfasst, welche dazu ausgelegt ist, das Funkmodul des Armbandcomputers zu aktivieren, sobald mittels der Erfassungseinrichtung der zumindest eine Vitalparameter des Benutzers erfasst wird, und das Funkmodul des Armbandcomputers zu deaktivieren, sobald mittels der Erfassungseinrichtung der zumindest eine Vitalparameter des Benutzers nicht mehr erfasst wird.

Der Erfindung liegt dabei die Erkenntnis zugrunde, dass dauerhaft aktive drahtlose Datenverbindungen üblicherweise relativ viel Energie verbrauchen. Bei am Arm tragbaren Armbandcomputern, beispielsweise in Form von Smartwatches, wird üblicherweise die Herstellung und Aufrechterhaltung einer drahtlosen Datenverbindung zwischen dem Armbandcomputer und einem anderen Objekt, insbesondere einer elektronischen Vorrichtung, oft nur dann benötigt, wenn der Armbandcomputer auch tatsächlich am Handgelenk getragen wird.

Daher ist es erfindungsgemäß vorgesehen, dass das Funkmodul nur dann aktiviert wird und in diesem aktivierten Zustand gehalten wird, solange mittels der Erfassungseinrichtung der zumindest eine Vitalparameter des Benutzers erfasst wird. Die Erfassungseinrichtung ist dabei dazu ausgebildet, den zumindest einen Vitalparameter des Benutzers zumindest solange zu erfassen, wie zumindest ein mit der Erfassungseinrichtung verbundener Teil des Armbandcomputers mit dem Arm des Benutzers in Kontakt steht. Sobald also der Armbandcomputer nicht mehr am Arm des Benutzers getragen wird, wird das Funkmodul des Armbandcomputers deaktiviert. Sobald der Armbandcomputer wieder am Arm des Benutzers angelegt und getragen wird, wird dies durch die Erfassungseinrichtung erfasst, da diese dazu ausgelegt ist, zumindest einen Vitalparameter des Benutzers zu erfassen. Sobald der Armbandcomputer wieder am Arm des Benutzers angelegt worden ist, wird also das Funkmodul wieder aktiviert. Dadurch, dass das Funkmodul des Armbandcomputers nur dann aktiv geschaltet wird, wenn der Armbandcomputer auch tatsächlich am Arm eines Benutzers getragen und somit benutzt wird, kann sehr viel Energie eingespart werden. Die Nutzungsdauer des Armbandcomputers kann dadurch erheblich verlängert werden.

Eine vorteilhafte Ausführungsform des Armbandcomputers sieht vor, dass die Erfassungseinrichtung einen Pulssensor aufweist, welcher dazu ausgelegt ist, einen Pulsschlag des Benutzers zu erfassen. Als Smartwatches ausgebildete Armbandcomputer werden üblicherweise am Handgelenk getragen. Mittels des Pulssensors kann dadurch auf besonders einfache Weise festgestellt werden, ob der Armbandcomputer gerade vom Benutzer angelegt worden oder abgelegt worden ist. Dadurch kann die Aktivierung und Deaktivierung des Funkmoduls des Armbandcomputers immer passgenau erfolgen, nämlich dann, wenn der Armbandcomputer am Arm des Benutzers angelegt bzw. abgelegt worden ist.

Eine weitere vorteilhafte Ausführungsform des Armbandcomputers sieht vor, dass die Erfassungseinrichtung einen Temperatursensor aufweist, welcher dazu ausgelegt ist, eine Hauttemperatur des Benutzers zu erfassen. In einem Speicher des Armbandcomputers kann beispielsweise eine Soll-Hauttemperatur von zum Beispiel 21 bis 35° C abgespeichert sein. Solange der Temperatursensor ermittelt, dass die ermittelte Temperatur mit dieser Soll-Temperatur zumindest im Wesentlichen übereinstimmt, wird davon ausgegangen, dass der Armbandcomputer am Arm des Benutzers getragen wird. Mittels des Temperatursensors kann unter Ermittlung der Hauttemperatur des Benutzers auf einfache Weise ebenfalls bestimmt werden, ob der Armbandcomputer gerade am Arm getragen wird oder nicht. Die Aktivierung und Deaktivierung des Funkmoduls des Armbandsensors kann daher ebenfalls besonders passgenau erfolgen.

Gemäß einer weiteren vorteilhaften Ausführungsform des Armbandcomputers ist es vorgesehen, dass das Funkmodul dazu ausgelegt ist, die drahtlose Verbindung in Form einer Bluetooth-Verbindung herzustellen und aufrechtzuerhalten. Der Aufbau und die Aufrechterhaltung einer Bluetooth-Verbindung benötigt im Vergleich mit anderen drahtlosen Verbindungen noch relativ wenig Energie, weist eine relativ geringe Störempfindlichkeit auf und erfordert eine relativ niedrige Sendeleistung.

Gemäß einer weiteren vorteilhaften Ausführungsform des Armbandcomputers ist es vorgesehen, dass das Funkmodul des Armbandcomputers dazu ausgelegt ist, eine Benutzerkennung über die drahtlose Datenverbindung an ein Funkmodul eines Kraftfahrzeugs zu senden. Der Armbandcomputer kann dadurch auf besonders einfache Weise beispielsweise zu Authentifizierungszwecken im Zusammenhang mit der Verwendung des Kraftfahrzeugs eingesetzt werden.

Das erfindungsgemäße Zugangssystem für ein Kraftfahrzeug umfasst den erfindungsgemäßen Armbandcomputer oder eine vorteilhafte Ausführungsform des erfindungsgemäßen Armbandcomputers, eine fahrzeugseitige Verriegelungseinrichtung zum Ver- und Entriegeln des Kraftfahrzeugs, ein fahrzeugseitiges Funkmodul und eine fahrzeugseitige biometrische Zugangsvorrichtung, welche dazu ausgelegt ist, die Verriegelungseinrichtung nur dann zum Verriegeln und/oder Entriegeln des Kraftfahrzeugs zu betätigen, wenn mittels der biometrischen Zugangsvorrichtung ein vorgegebenes biometrisches Merkmal einer Person erfasst und mittels des Funkmoduls des Armbandcomputers über eine drahtlose Verbindung eine der Person zugeordnete Benutzererkennung an das fahrzeugseitige Funkmodul übertragen worden ist. Durch die Integration des erfindungsgemäßen Armbandcomputers in das Zugangssystem für das Kraftfahrzeug kann ein besonders sicheres Zugangssystem bereitgestellt werden. Vorzugsweise umfasst die biometrische Zugangsvorrichtung einen Handvenenscanner. Bei der Benutzung eines biometrischen Zugangssystems, wie zum Beispiel einem Handvenenscanner, muss dem Kraftfahrzeug üblicherweise die Identität der Person bekannt sein, bevor diese sich am Kraftfahrzeug authentifizieren kann, damit eine Verifikation vorgenommen werden kann. Zudem wird ein derartiges Zugangssystem üblicherweise auch nur aktiviert, wenn eine authentifizierungsberechtigte Person an das Fahrzeug herantritt und nicht nur irgend ein vorbeilaufender Passant. Die Identifikation unter Verwendung des erfindungsgemäßen Armbandcomputers erfolgt dabei vorzugsweise über Bluetooth. Ein Nachteil einer derartigen Identifikation ist allerdings, dass die Position der Person nicht genau bestimmt werden kann. Somit kann nicht unterschieden werden, ob sich die Person gerade im Kraftfahrzeug oder außerhalb vom Kraftfahrzeug befindet. Soll das Kraftfahrzeug nun über den Handvenenscanner abgeschlossen werden, ist es besonders vorteilhaft, wenn sichergestellt sein kann, dass sich der Armbandcomputer nicht innerhalb des Kraftfahrzeugs befindet. Da die drahtlose Verbindung, vorzugsweise in Form der Bluetooth-Verbindung, deaktiviert wird, sobald der Armbandcomputer abgenommen wird, kann das Kraftfahrzeug gar nicht mehr abgeschlossen werden, wenn ein Fahrzeuginsasse den Armbandcomputer vor dem Aussteigen aus dem Kraftfahrzeug abgenommen hat. Ein versehentliches Aussperren des Fahrzeuginsassen kann dadurch effektiv verhindert werden.

Bei einem Verfahren zum Betreiben eines Armbandcomputers zum Tragen an einem Arm eines Benutzers wird mittels einer Erfassungseinrichtung des Armbandcomputers zumindest ein Vitalparameter des Benutzers erfasst und mittels eines Funkmoduls des Armbandcomputers eine drahtlose Verbindung mit einem Funkmodul eines anderen Objekts hergestellt und aufrechterhalten, solange das Funkmodul des Armbandcomputers aktiviert ist. Das Verfahren zeichnet sich dabei dadurch aus, dass mittels einer Steuereinrichtung des Armbandcomputers das Funkmodul des Armbandcomputers aktiviert wird, sobald mittels der Erfassungseinrichtung der zumindest eine Vitalparameter des Benutzers erfasst wird, und das Funkmodul des Armbandcomputers mittels der Steuereinrichtung deaktiviert wird, sobald mittels der Erfassungseinrichtung der zumindest eine Vitalparameter des Benutzers nicht mehr erfasst wird.

Weitere Vorteile, Merkmale und Einzelheiten der Erfindung ergeben sich aus der nachfolgenden Beschreibung eines bevorzugten Ausführungsbeispiels sowie anhand der Zeichnung. Die vorstehend in der Beschreibung genannten Merkmale und Merkmalskombinationen sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen oder in Alleinstellung verwendbar, ohne den Rahmen der Erfindung zu verlassen.

Die Zeichnung zeigt in:
- Fig. 1: einen als Smartwatch ausgebildeten Armbandcomputer zum Tragen an einem Arm eines Benutzer;
- Fig. 2: eine schematische Darstellung der Smartwatch, welche ein Funkmodul, eine Steuereinrichtung und eine Erfassungseinrichtung zur Pulserfassung auffasst; und in
- Fig. 3: eine schematische Darstellung eines Kraftfahrzeugs, welches eine Verriegelungseinrichtung zum Ver- und Entriegeln des Kraftfahrzeugs, ein Funkmodul und eine biometrische Zugangsvorrichtung aufweist.

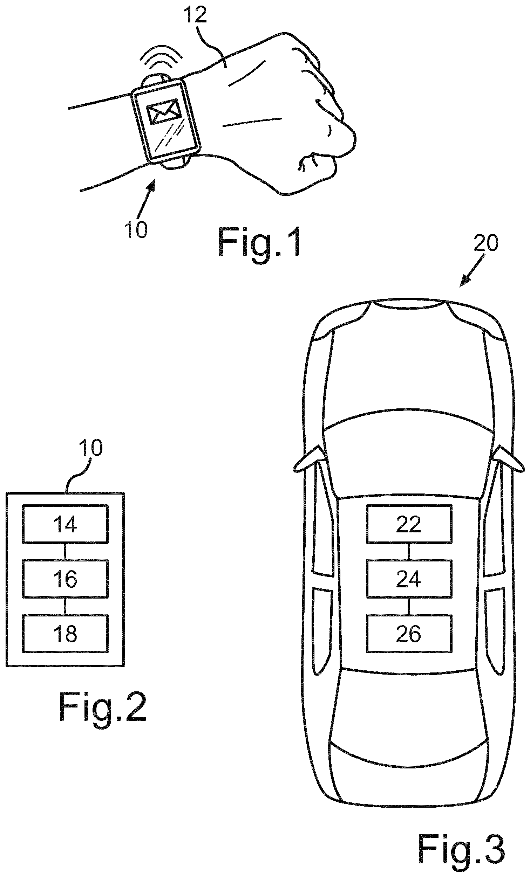

Ein als Smartwatch 10 ausgebildeter Armbandcomputer zum Tragen an einem Arm 12 eines Benutzers ist in einer Perspektivansicht in Fig. 1 gezeigt. Bei der Smartwatch handelt es sich um eine Armbanduhr, die zusätzlich über Sensoren, Aktuatoren, wie beispielsweise einen Vibrationsmotor und dergleichen, sowie zusätzliche Computerfunktionalitäten und Konnektivitätsfunktionen verfügt. Neben der reinen Uhrzeitanzeige kann die Smartwatch 10 eine Vielzahl weiterer Informationen darstellen und lässt sich über zusätzlich installierbare Programme, sogenannte Apps, vom Anwender individuell mit neuen Funktionen aufrüsten.

In Fig. 2 ist die Smartwatch 10 in einer schematischen Darstellung gezeigt. Die Smartwatch 10 umfasst ein Funkmodul 14, eine Steuereinrichtung 16 und eine Erfassungseinrichtung 18. Die Erfassungseinrichtung 18 ist dazu ausgelegt, einen Pulsschlag des Benutzers zu erfassen, sobald die Smartwatch 10 am Arm 12 des Benutzers angelegt worden ist. Dafür umfasst die Erfassungseinrichtung 18 zumindest einen Pulssensor.

Das Funkmodul 14 ist im aktivierten Zustand dazu ausgelegt, eine drahtlose Verbindung mit einem Funkmodul eines anderen Objekts herzustellen und aufrechtzuerhalten. Bei dem Funkmodul 14 handelt es sich um ein Bluetooth-Modul, welches dazu ausgelegt ist, die besagte drahtlose Verbindung in Form einer Bluetooth-Verbindung herzustellen und aufrechtzuerhalten. Die Steuereinrichtung ist dazu ausgelegt, das Funkmodul 14 der Smartwatch 10 zu aktivieren, sobald mittels der Erfassungseinrichtung 18 ein Pulsschlag des Benutzers, der die Smartwatch 10 am Arm 12 trägt, erfasst wird. Die Steuereinrichtung 16 ist zudem dazu ausgelegt, das Funkmodul 14 der Smartwatch 10 zu deaktivieren, sobald mittels der Erfassungseinrichtung 18 kein Pulsschlag mehr erfasst wird. Nimmt der Benutzer also die Smartwatch 10 von seinem Arm 12 ab, kann die Erfassungseinrichtung 18 keinen Pulsschlag mehr feststellen. Infolgedessen deaktiviert die Steuereinrichtung 16 das Funkmodul 14, wodurch das als Bluetoothadapter dienende Funkmodul 14 deaktiviert und sämtliche zuvor noch aktiven Verbindungen gekappt werden. Sobald der Benutzer die Smartwatch 10 wieder an seinem Arm 12 anlegt, erfasst die Erfassungseinrichtung 18 den Pulsschlag des Benutzers, infolgedessen das Funkmodul 14 wieder aktiviert wird.

In Fig. 3 ist ein Kraftfahrzeug 20 in einer schematischen Draufsicht gezeigt. Das Kraftfahrzeug 20 umfasst eine fahrzeugseitige Verriegelungseinrichtung 22 zum Ver- und Entriegeln des Kraftfahrzeugs 20. Darüber hinaus umfasst das Kraftfahrzeug 20 eine fahrzeugseitige biometrische Zugangsvorrichtung 24 und ein fahrzeugseitiges Funkmodul 26. Die Smartwatch 10 kann über ihr Funkmodul 14 eine Bluetooth-Verbindung mit dem Funkmodul 26 des Kraftfahrzeugs 10 aufbauen und dann eine Benutzerkennung an das fahrzeugseitige Funkmodul 26 übertragen. Die Smartwatch 10, die fahrzeugseitige Verriegelungseinrichtung 22, die fahrzeugseitige biometrische Zugangsvorrichtung 24 und das fahrzeugseitige Funkmodul 26 bilden zusammen ein Zugangssystem für das Kraftfahrzeug 20.

Bei der biometrischen Zugangsvorrichtung 24 handelt es sich um einen Handvenenscanner, mittels welchem sich ein Benutzer zum Ver- und Entriegeln des Kraftfahrzeugs 20 authentifizieren kann.

Die biometrische Zugangsvorrichtung 24 ist dazu ausgelegt, die Verriegelungseinrichtung 22 nur dann zum Verriegeln und/oder Entriegeln des Kraftfahrzeugs 20 zu betätigen, wenn mittels der biometrischen Zugangsvorrichtung 24 ein vorgegebenes biometrisches Merkmal einer Person erfasst und mittels des Funkmoduls 14 der Smartwatch 10 über eine drahtlose Verbindung, vorzugsweise über Bluetooth, eine der Person zugeordnete Benutzerkennung an das fahrzeugseitige Funkmodul 26 übertragen worden ist.

Möchte eine Person das Kraftfahrzeug 20 von außen abschließen, so muss sie sich zum einen einer Handvenenscannung zur Authentifizierung unterziehen und zum anderen muss von der Smartwatch 10 am fahrzeugseitigen Funkmodul 26 eine entsprechenden Benutzerkennung eingegangen sein.

Sollte die Person die Smartwatch 10 während der Fahrt mit dem Kraftfahrzeug 20 abgelegt haben, so wird das Funkmodul 14 der Smartwatch 10 deaktiviert. Steigt der Fahrzeuginsasse nun aus dem Kraftfahrzeug 20 aus und möchte dieses durch einen Handvenenerkennung absperren, wird das Kraftfahrzeug nicht abgesperrt, da das Funkmodul 14 der Smartwatch 10 deaktiviert ist und somit die Benutzerkennung nicht an das fahrzeugseitige Funkmodul 26 übertragen werden kann. Die Person kann sich also bei im Kraftfahrzeug 20 vergessener Smartwatch 10 nicht versehentlich aus dem Kraftfahrzeug 20 aussperren.

## Patentansprüche

1. Zugangssystem für ein Kraftfahrzeug (20), mit
- einem Armbandcomputer (10) zum Tragen an einem Arm (12) eines Benutzers, umfassend eine Erfassungseinrichtung (18), welche dazu ausgelegt ist, zumindest einen Vitalparameter des Benutzers zu erfassen, ein Funkmodul (14), welches im aktivierten Zustand dazu ausgelegt ist, eine drahtlose Datenverbindung mit einem Funkmodul (26) eines anderen Objekts (20) herzustellen und aufrechtzuerhalten, wobei der Armbandcomputer (10) eine Steuereinrichtung (16) umfasst, welche dazu ausgelegt ist, das Funkmodul (14) des Armbandcomputers (10) zu aktivieren, sobald mittels der Erfassungseinrichtung (18) der zumindest eine Vitalparameter des Benutzers erfasst wird, und das Funkmodul (14) des Armbandcomputers (10) zu deaktivieren, sobald mittels der Erfassungseinrichtung (18) der zumindest eine Vitalparameter des Benutzers nicht mehr erfasst wird;
- einer fahrzeugseitigen Verriegelungseineinrichtung (22) zum Ver- und Entriegeln des Kraftfahrzeugs (20),
- einem fahrzeugseitigen Funkmodul (26) und einer fahrzeugseitigen biometrischen Zugangsvorrichtung (24), welche dazu ausgelegt ist, die Verriegelungseineinrichtung (22) nur dann zum Verriegeln und/oder Entriegeln des Kraftfahrzeugs (20) zu betätigen, wenn mittels der biometrischen Zugangsvorrichtung (24) ein vorgegebenes biometrisches Merkmal einer Person erfasst und mittels des Funkmoduls (14) des Armbandcomputers (10) über eine drahtlose Verbindung eine der Person zugeordnete Benutzerkennung an das fahrzeugseitige Funkmodul (26) übertragen worden ist.

2. Zugangssystem nach Anspruch 1,
**dadurch gekennzeichnet, dass**
die biometrische Zugangsvorrichtung (24) einen Handvenenscanner umfasst.

3. Zugangssystem nach Anspruch 1 oder 2,
**dadurch gekennzeichnet, dass**
die Erfassungseinrichtung (18) einen Pulssensor aufweist, welcher dazu ausgelegt ist, einen Pulsschlag des Benutzers zu erfassen.

4. Zugangssystem nach einem der vorhergehenden Ansprüche,
**dadurch gekennzeichnet, dass**
die Erfassungseinrichtung (18) einen Temperatursensor aufweist, welcher dazu ausgelegt ist, eine Hauttemperatur des Benutzers zu erfassen.

5. Zugangssystem nach einem der vorhergehenden Ansprüche,
**dadurch gekennzeichnet, dass**
das Funkmodul (14) des Armbandcomputers (10) dazu ausgelegt ist, die drahtlose Verbindung in Form einer Bluetooth-Verbindung herzustellen und aufrechtzuerhalten.

6. Zugangssystem nach einem der vorhergehenden Ansprüche,
**dadurch gekennzeichnet, dass**
das Funkmodul (14) des Armbandcomputers (10) dazu ausgelegt ist, eine Benutzerkennung über die drahtlose Datenverbindung an ein Funkmodul (26) eines Kraftfahrzeugs (20) zu senden.

## Claims

1. Access system for a motor vehicle (20), having
- an armband computer (10) to be worn on an arm (12) of a user, comprising a detection device (18) configured to detect at least one vital parameter of the user, a radio module (14) configured in the activated state to establish and maintain a wireless data connection to a radio module (26) of another object (20), the armband computer (10) comprising a control device (16) configured to activate the radio module (14) of the armband computer (10) once the at least one vital parameter of the user is detected by means of the detection device (18), and deactivate the radio module (14) of the armband computer (10) once the at least one vital parameter of the user is no longer detected by means of the detection device (18);
- a locking device (22) on the side of the vehicle for locking and unlocking the motor vehicle (20),
- a radio module (26) on the side of the vehicle and a biometric access device (24) on the side of the vehicle configured to actuate the locking device (22) on the side of the vehicle for locking or unlocking the motor vehicle (20) only when a predefined biometric feature of a person has been detected by means of the biometric access device (24) and a user identification associated with the person has been transmitted to the radio module (26) on the side of the vehicle by means of the radio module (14) of the armband computer (10) via a wireless connection.

2. Access system according to claim 1,
**characterised in that**
the biometric access device (24) comprises a hand vein scanner.

3. Access system according to claim 1 or 2,
**characterised in that**
the detection device (18) has a pulse sensor configured to detect a pulse of the user.

4. Access system according to any of the preceding claims,
**characterised in that**
the detection device (18) has a temperature sensor configured to detect a skin temperature of the user.

5. Access system according to any of the preceding claims,
**characterised in that**
the radio module (14) of the armband computer (10) is configured to establish and maintain the wireless connection in the form of a Bluetooth connection.

6. Access system according to any of the preceding claims,
**characterised in that**
the radio module (14) of the armband computer (10) is configured to transmit a user identification via the wireless data connection to a radio module (26) of a motor vehicle (20).

## Revendications

1. Système d'accès pour un véhicule automobile (20), avec
- une montre-ordinateur (10) destinée à être portée au niveau d'un bras (12) d'un utilisateur, comprenant un dispositif de détection (18), qui est conçu afin de détecter au moins un paramètre vital de l'utilisateur, un module radio (14) qui est conçu dans l'état activé afin d'établir et de maintenir une liaison de données sans fil avec un module radio (26) d'un autre objet (20), dans lequel la montre-ordinateur (10) comprend un dispositif de commande (16) qui est conçu afin d'activer le module radio (14) de la montre-ordinateur (10) dès qu'au moyen du dispositif de détection (18) l'au moins un paramètre vital de l'utilisateur est détecté, et de désactiver le module radio (14) de la montre-ordinateur (10) dès qu'au moyen du dispositif de détection (18) l'au moins un paramètre vital de l'utilisateur n'est plus détecté ;
- un dispositif de verrouillage (22) côté véhicule pour le verrouillage et le déverrouillage du véhicule automobile (20),
- un module radio (26) côté véhicule et un dispositif d'accès biométrique (24) côté véhicule qui est conçu afin d'actionner le dispositif de verrouillage (22) seulement pour le verrouillage et/ou le déverrouillage du véhicule automobile (20) lorsqu'au moyen du dispositif d'accès biométrique (24) une caractéristique biométrique prescrite d'une personne a été détectée et au moyen du module radio (14) de la montre-ordinateur (10) par le biais d'une liaison sans fil une reconnaissance utilisateur associée à la personne a été transmise au module radio (26) côté véhicule.

2. Système d'accès selon la revendication 1,
**caractérisé en ce que**
le dispositif d'accès biométrique (24) comprend un scanner des veines de la main.

3. Système d'accès selon la revendication 1 ou 2,
**caractérisé en ce que**
le dispositif de détection (18) présente un capteur de pulsations qui est conçu afin de détecter un pouls de l'utilisateur.

4. Système d'accès selon l'une quelconque des revendications précédentes,
**caractérisé en ce que**
le dispositif de détection (18) présente un capteur de température qui est conçu afin de détecter une température de la peau de l'utilisateur.

5. Système d'accès selon l'une quelconque des revendications précédentes,
**caractérisé en ce que**
le module radio (14) de la montre-ordinateur (10) est conçu afin d'établir et de maintenir la liaison sans fil sous la forme d'une liaison Bluetooth.

6. Système d'accès selon l'une quelconque des revendications précédentes,
**caractérisé en ce que**
le module radio (14) de la montre-ordinateur (10) est conçu afin d'envoyer une reconnaissance utilisateur par le biais de la liaison radio sans fil à un module radio (26) d'un véhicule automobile (20).
